# EUROPEAN PATENT APPLICATION

(11) **EP 2 875 803 A1**
(43) Date of publication of application: **27.05.2015**
(21) Application number: 13194365.6
(22) Date of filing: 26.11.2013
(51) Int. Cl.: A61K 8/34, A61Q 17/04, A61Q 19/02, A61K 8/898, A61K 8/06

(54) **Polyol-in-oil-emulsions for dermal delivery**

(71) Applicant: OTC GmbH, 46047 Oberhausen (DE)
(72) Inventor: Dahms, Gerd, 47138 Duisburg (DE)
(74) Representative: Michalski Hüttermann & Partner Patentanwälte mbB

(57) **Abstract**

The invention relates to an optically transparent polyol-in-oil-composition, wherein the composition comprises a) ≥ 40 weight-% and ≤ 90 weight-% of at least one low molecular weight polyol, b) ≥ 1,0 weight-% and ≤ 25 weight-% of at least one cosmetic or therapeutic active, c) ≥ 1,0 weight-% and ≤ 10 weight-% of at least one polyether-free emulsifier d) ≥ 2,0 weight-% and ≤ 25 weight-% of at least one oil component, wherein the emulsion comprises emulsifier coated droplets in a size range of ≥ 20 nm and ≤ 1000.

## Description

### FIELD OF THE INVENTION

The invention relates to an optically transparent polyol-in-oil-composition, wherein the composition comprises a) ≥ 40 weight-% and ≤ 90 weight-% of at least one low molecular weight polyol, b) ≥ 1,0 weight-% and ≤ 25 weight-% of at least one cosmetic or therapeutic active, c) ≥ 1,0 weight-% and ≤ 10 weight-% of at least one polyether-free emulsifier d) ≥ 2,0 weight-% and ≤ 25 weight-% of at least one oil component, wherein the emulsion comprises emulsifier coated droplets in a size range of ≥ 20 nm and ≤ 1000 nm.

### BACKGROUND OF THE INVENTION

Sophisticated galenical formulations have evolved in the last decades in the pharmacological, dermatological and cosmetic field, which are able to tailor the delivery properties of highly active ingredients to the special requirements of the designated application. Due to ease of application, efficacy, skin feel and cost reasons mostly water containing emulsion type formulations are used, which comprise the vast majority of the available products in the above mentioned markets. Nevertheless, due to the water activity in such galenic systems it might be difficult in certain cases to ensure an ample stability of the formulation. This is especially true, if active components have to be incorporated, which do show strong tendencies of hydrolysis or any other kind of water triggered degradation.

In order to overcome such kind of stability issues several different approaches have been developed for dermatological or cosmetic applications, claiming to protect labile ingredients from degradation. Therefore, the active concentration is kept constant during storage and consequently the efficacy of the overall formulation should be maintained.

One possible strategy to protect unstable ingredients is for instance given by JP2009215298 A, wherein a composition in the form of an oil-in-water type emulsion is described, including an ascorbic acid compound or a salicylic acid compound; an additional surface active agent selected from an ester of a fatty acid and polyethylene glycol, an ester of a 16-22C fatty acid and sorbitan, and an ester of a 16-22C fatty acid and glycerol; and a condensation polymer of ethylene oxide and propylene oxide.

Another approach is described in EP2116223 A1, disclosing a dispersion composition including a carotenoid-containing oily components, the dispersion composition being obtained by mixing an aqueous dispersion containing emulsion particles containing the carotenoid-containing oily component and a phospholipid or a derivative thereof with an aqueous composition containing ascorbic acid or a derivative thereof and an oily component that is 20% by mass or less relative to the mass of the whole dispersion composition, and the dispersion composition having emulsion particles having an average particle diameter of 200 nm or less. The dispersion composition being obtained by mixing an aqueous dispersion containing emulsion particles containing a carotenoid-containing oily component and a phospholipid or a derivative thereof with an aqueous composition containing ascorbic acid or a derivative thereof and a pH adjusting agent, and the dispersion composition having a pH value in the range of 5 to 7.5; and a cosmetic preparation for skin care including one or other of the dispersion compositions.

Furthermore, JP2006117534 A describes an emulsified cosmetic having an excellent feel in use and stability with time in a bleaching cosmetic comprising ascorbic acid glucoside as an active ingredient. The emulsified cosmetic comprises (a) ascorbic acid glucoside, (b) a polyacrylamide, (c) behenyl alcohol and (d) a nonionic surfactant having >=10 HLB and has a viscosity of 2,000-20,000 mPa s at 25° C.

Nevertheless, above mentioned strategies either comprises the active component in a stabilized form or water is present in the composition in considerable amounts from the start of processing. In the latter case this may lead to the formation of degradation products in the course of processing and/or storage and in the first case the efficacy of the active ingredient may be reduced due to the use of protective groups.

Taking into account that a lot of beneficial actives are only sparingly soluble in the common hydrophilic and hydrophobic emulsion-type environments and, as stated above, the stability or the efficacy of the active is disadvantageously influenced by the presence of water or chemical modification of the active, there is still the need to provide further dermatological acceptable, easily applicable galenic formulations, which are able to protect sensitive ingredients from degradation during storage.

It was therefore an obj ect of this invention to provide such a composition without one or more of the disadvantages of the prior art, i.e. a composition which is capable of dissolving significant amounts of hydrophilic or hydrophobic actives, having a comfortable feel when applied to body surfaces, exhibiting excellent stability and penetration behaviour and being easily processable.

### SUMMARY OF THE INVENTION

Surprisingly it has been found, that an optically transparent polyol-in-oil-composition, characterised in that the composition comprises a) ≥ 40 weight-% and ≤ 90 weight-% of at least one low molecular weight polyol, b) ≥ 1,0 weight-% and ≤ 25 weight-% of at least one cosmetic or therapeutic active, c) ≥ 1,0 weight-% and ≤ 10 weight-% of at least one polyether-free emulsifier d) ≥ 2,0 weight-% and ≤ 25 weight-% of at least one oil component, wherein the emulsion comprises emulsifier coated droplets in a size range of ≥ 20 nm and ≤ 1000 nm, can suitably be used to homogeneously dissolve a wide variety of different cosmetic or dermatological actives at high concentrations and provide a safe environment for enhanced active stability compared to state of the art aqueous emulsions. In addition, the inventive composition is also able to homogeneously incorporate sparingly soluble ingredients, thus forming an optically transparent emulsion. Due to the absence of water the stability of hydrolysis sensitive ingredients is enhanced and the activity of the cosmetic or therapeutic active is maintained even at higher storage temperatures. The inventive composition is very skin-friendly and yields a good penetration profile of hydrophilic and hydrophobic actives. Without being bound by the theory the penetration of hydrophobic ingredients may be enhanced due to the reduction of thermodynamic activity of hydrophobic actives in the hydrophilic composition by penetration into the hydrophobic lipid mantle of the skin. A good penetration of hydrophilic actives may be achieved due to the inventive emulsifier coated droplets size, being small enough to yield a good contact of the emulsifier coated droplets to the skin. Furthermore, due to the presence of polyether-free emulsifier unwanted peroxo degradation products, which may be generated by auto-oxidation of polyether comprising emulsifiers like PEGs or PPGs, are avoided during storage. Such degradation products may be either highly aggressive to dermal structures or might result in an unpleasant odor of the formulation. In addition, the overall composition is easily processable and very tolerant to the inclusion of further functional compounds like scents, ph-adjusters, viscosity adjuster, pigments etc.. Furthermore it is advantageous that above the critical phase-volume ratio the polyol-in-oil-compositions do form a lyotropic liquid crystalline phase exhibiting inverse cubic structures. Without being bound by the theory such phase can help to stabilize the composition thermodynamically and effectively shield the active.

The polyol-in-oil-compositions according to the invention are optically transparent, i.e. the composition is either optically clear or only slightly opaque. This is in contrast to state of the art polyol-in-oil-compositions, which are, due to the presence of un-dissolved actives or larger emulsifier coated droplets, usually non-transparent emulsions.

A low molecular weight polyol in the sense of this invention comprise a molecular weight below 500 g/mol, preferably below 400 g/mol and more preferably below 300 g/mol and more than one hydroxyl moiety. Such kinds of polyols have the right size to penetrate within the application time into the skin or hair and hence contribute to the cosmetic efficacy and, in addition, are also able to stabilize the cosmetic active. These polyols comprise more than one alcohol moiety and are cyclic, linear and branched polyols like propylene-glycol, glycerine, poly-glycerine, sorbitol, mannitol, threitol, arabitol, xylitol, ribitol, butylene-glycole, pentylene-glycol, hexylene-glycol and dimethyl isosorbide. The inventive polyol-in-oil-composition may also be achieved by using polyol mixtures. Especially preferred are non-ethoxylated polyols comprising more than one alcohol moiety. Such polyols are less prone to auto-oxidation and therefore are able to enhance the chemical stability of the composition. Especially preferred polyols may for instance be propylene-glycol, glycerine, sorbitol, mannitol, threitol, arabitol, xylitol, ribitol, butylene-glycole, pentylene-glycol, hexylene-glycol and dimethyl isosorbide.

The cosmetic or therapeutic active can be selected from cosmetically or therapeutically active agents known to the skilled in the art which are suitable for percutaneous applications. I.e. including a sufficient therapeutic potential from a broad spectrum for different indications and fields of use, in particular anti-acne agents, antimicrobial agents, antiperspirants, astringents, deodorants, hair-removing agents, conditioning agents for the skin, skin-smoothing agents, agents for increasing the hydration of the skin, for example urea, sun protection agents, keratolytics, radical acceptors for free radicals, antiseptic active ingredients, active ingredients for treating the signs of skin ageing and/or agents which modulate the differentiation and/or proliferation and/or pigmentation of the skin, vitamins such as vitamin C, active ingredients having a stimulating side-effect, such as alpha-hydroxy acids, β-hydroxy acids, alpha-keto acids, β-keto acids, retinoids (retinol, retinal, retinic acid), anthralins (dioxyanthranol), anthranoids, peroxides (especially benzoyl peroxide), minoxidil, lithium salts, antimetabolites, vitamin D and its derivatives; catechols, flavonoids, ceramides, evening primrose oil, plant lecithins (e.g. soya lecithin), sphingolipids/ceramides isolated from plants, polyunsaturated fatty acids, essential fatty acids (e.g. gamma-linolenic acid), enzymes, coenzymes, enzyme inhibitors, hydrating agents, skin-calming agents, detergents or foam-forming agents, and inorganic or synthetic delustering fillers, abrasive agents. The inventive polyol-in-oil-composition is also achieved by using mixtures of different actives.

According to the invention polyether-free emulsifier are used to form the inventive polyol-in-oil-composition. Suitable polyether-free emulsifier, which can be used for cosmetic purpose can for instance be found in the Handbook of Green Chemicals by Michael Ash and Irene Ash, 2ed, June 2004, ISBN-13: 978-1890595791. In general, glycerine-ester, sorbitan-ester, sorbitol-ester, fatty alcohols, propylene-glycolester, alkyl-glucosit-ester, sugar-ester, lecithine, stearic acid, myristic acid, lauric acid, succinates, amides or ethanolamides of fatty acids and silicon emulsifiers may be used. It is also possible to use mixtures of different emulsifiers-types. Again, due to stability reasons the non-ethoxylated silicon emulsifier may especially be used.

Preferably the emulsifiers are selected from the group of amino-functional emulsifier. Such emulsifier can effectively form polyol-in-oil-emulsions and are known as very skin friendly emulsifiers.

The oil component can be selected from all kinds of cosmetically or pharmacologically acceptable oils. Examples for oils suitable for cosmetic purposes are mentioned, for example, in CTFA transactions, Cosmetic Ingredient Handbook, 1st Edition, 1988, The Cosmetic, Toiletry and Fragrance Association, Inc., Washington. Suitable examples, which can be used in the inventive polyol-in-oil-compositions can for instance be mineral oils, such as paraffin oils or vaseline oils, silicone oils, vegetable oils such as coconut oil, sweet almond oil, apricot oil, corn oil, jojoba oil, olive oil, avocado oil, sesame oil, palm oil, eucalyptus oil, rosemary oil, lavender oil, pine oil, thyme oil, mint oil, cardamom oil, orange-blossom oil, soybean oil, bran oil, rice oil, rapeseed oil and castor oil, fatty-acid esters, esters of fatty alcohols such as triglycerides.

In addition, also silicone oils can be used in the present invention, either as volatile and/or non-volatile silicon oils. Preferred silicone oils are non-volatile silicone oils known with their INCI name as dimethicone and dimethiconol. Volatile silicone oils such as cyclomethicones may be used in combination with non-volatile silicones and/or other wax and/or oils mentioned above. Commercially, they are available from various companies, for example Dow Coming with the known DC series, Wacker Chemie and Toray silicones. All commercially available non volatile silicones are suitable in the compositions of the present invention. Furthermore, arylated silicones comprising at least one aryl group in its molecule such as phenyl methicone, phenyl trimethicone, diphenyl dimethicone, diphenylsiloxy phenyl trimethicone, tetramethyl tetraphenyl trisiloxane, triphenyl trimethicone, tetramethyl tetraphenyl trisiloxane and trimethyl pentaphenyl trisiloxane can be advantageously comprised in the compositions of the present invention.

Especially the use of non-polar oils like in silicon oils, cyclomethicone, paraffin oils, isoparaf fine oils, vaseline or squalane do result in the formation of very stable emulsifier coated droplets and therefore such group of non polar-oils can preferably be used for building the inventive polyol-in-oil-compositions.

The inventive composition comprises emulsifier coated droplets in a size range of ≥ 20 nm and ≤ 1000 nm. Preferably the size range of the emulsifier coated droplets can be in a range of ≥ 50 nm and ≤ 600 nm and additionally preferred of ≥ 100 nm and ≤ 500 nm. Such size range enables a stable formation of the emulsion without any form of coalescence of the emulsifier coated droplets and is a pre-requisite that the polyol-in-oil-composition is optically clear. The size of the emulsifier coated droplets can be assessed by scattering techniques like laser light scattering. Here especially MALLS (multi angle laser light scattering) can be used to determine the size distribution of the emulsifier coated droplets.

In an additional aspect of the invention the emulsifier coated droplets size distribution is of gauss type. Lorentz or Cauchy-type size distributions are not preferred, because this kind of size distribution might lead to stability problem during storage of the polyol-in-oil-composition.

In a preferred aspect of the invention the water content of the composition can be below ≤ 1 weight%, preferably ≤ 0,5 weight% and even more preferred ≤ 0,1 weight%. Low water contents are especially suitable if a composition is required which exhibits self preserving properties. Here no additional preservative has to be added. Composition comprising a low water content might also be considered as concentrates, which can be adjusted with respect to a suitable viscosity or active range right before the application by adding water, thus forming polyol-in-oil-in-water (POW) compositions.

In a further embodiment of the invention the composition can be formulated in a way that it is essentially free of preservatives. The high polyol content of the formulation might lead to a significantly reduced water activity in the composition, which in turn results in a self preserving system.

### DETAILED DESCRIPTION OF THE PREFERRED EMBODIMENTS

Within a further object of the inventive composition the emulsifier is an amino-functional silicon-derivative. Especially amino-functional silicon-derivatives have proven to be very suitable for an easy processing of the polyol-in-oil-composition. Furthermore, such emulsifiers are very skin friendly and very effective even at low concentrations. Without being bound by the theory this is especially due to the combination of the silicon-backbone and the amino-functional moiety. Suitable amino-functional emulsifier may be selected from the Wacker Belsil^{®} ADM-range or Clariant SilCare® Silicone WSI-range. In a preferred embodiment of the invention especially Amidomethicone can be used.

In another preferred aspect of the inventive composition the emulsifier is amodimethicone glycerocarbamate. Surprisingly it has been found, that especially amodimethicone glycerocarbamate is effectively able to form stable polyol-in-oil-compositions, which do show an exceptional high stability. Additionally, this emulsifier is able to assist in the dispersion process of the active. Without being bound by the theory this might be caused by the high molecular weight of this ethylene oxide-free silicone emulsifier. This emulsifier is liquid at ambient temperatures and can additionally be used over a broad pH range, exhibiting only a medium viscosity. These features ease the processing and handling. Further it is preferred, that the emulsifier amodimethicone glycerocarbamate is the only emulsifier in the system, resulting in a polyether-free composition, exhibiting good storage stability and easy handling.

An additional embodiment according to the invention comprises a composition, wherein additionally a low molecular weight alcohol is present in an amount of ≥ 0,5 weight-% and ≤ 15 weight%. It has surprisingly been found that the skin feel of the inventive polyol-in-oil-composition can drastically been altered by the addition of monovalent alcohols. Different skin sensation like cooling or skin feel after the application can thus be tailored. Lower molecular weight alcohols which may suitably be used in the present invention are monovalent aliphatic alcohols having 1 to 4 carbon atoms and one OH-moiety. Specific examples may include ethanol, propanol, isopropanol, isobutyl alcohol, and t-butyl alcohol. Preferably ethanol can be used. Furthermore, the low molecular weight alcohol is effectively able to change the penetration behavior of certain actives. In the case that actives, which hardly penetrate through the lipid layer of the skin, shall be delivered to deeper skin layers, this content of low molecular weight alcohol might be favorably used to increase the dermal penetration of the active. Suitable effects on the skin feel can additionally be achieved at alcohol concentrations of ≥ 1,5 weight-% and ≤ 10 weight% and preferably ≥ 3,0 weight-% and ≤ 7,5 weight%.

A preferred characteristic of the invention disclose a composition, wherein additionally polyvinylpyrrolidone and/or polyvinylcaprolactam is present in an amount of ≥ 0,5 weight-% and ≤ 15 weight-%. The incorporation of polyvinylpyrrolidone and/or polyvinylcaprolactam might result in a further stabilization of the emulsion and the active. Furthermore, polyvinylpyrrolidone and/or polyvinylcaprolactam of different molecular weights might be able to effectively increase the penetration of the active. Therefore, polyvinylpyrrolidones and/or polyvinylcaprolactam in a molecular weight range of ≥ 500 Da and ≤ 2 x10⁶ Da, preferably of ≥ 1000 Da and ≤ 500.000 Da may be used to increase the release and penetration profile of the active. In certain cases also polyvinylpyrrolidone and/or polyvinylcaprolactam concentrations of ≥ 1,0 weight-% and ≤ 12 weight-% and ≥ 5,0 weight-% and ≤ 10 weight-% may suitably be incorporated in the polyol-in-oil-composition. It is also possible to use mixtures of polyvinylpyrrolidone and/or polyvinylcaprolactam.

A preferred characteristic of the invention disclose a composition, wherein additionally a polyvinylcaprolactam is present in an amount of ≥ 0,5 weight-% and ≤ 15 weight-%. The incorporation of polyvinylcaprolactam might result in a further stabilization of the emulsion and the active. Furthermore, polyvinylcaprolactam of different molecular weights might be able to effectively increase the penetration of the active. Therefore, polyvinylcaprolactam in a molecular weight range of ≥ 500 Da and ≤ 2 x10⁶ Da, preferably of ≥ 1000 Da and ≤ 500.000 Da may be used to increase the release and penetration profile of the active. In certain cases also polyvinylcaprolactam concentrations of ≥ 1,0 weight-% and ≤ 12 weight-% and ≥ 5,0 weight-% and ≤ 10 weight-% may suitably be incorporated in the polyol-in-oil-composition. Furthermore, also mixtures of polyvinylcaprolactam and polyvinylpyrrolidone may suitably be used in the inventive composition.

Additionally, the inventive composition may comprise a cosmetic or therapeutic active which is selected from the group comprising ascorbic acid, tocopherol, retinol, beta-carotene, polyphenols, trolox, quercetin, rutin, myricetin, kaempferol, catechin, epigallocatechin, epicatechin, resveratrol, red wine extract, white wine extract, grape extract, black tea extract, green tea extract, white tea extract. The above mentioned group of actives can suitably be incorporated into the inventive polyol-in-oil-composition and yield clear polyol-in-oil-composition. All members of the group exhibit an LDL-oxidation inhibition value IC₅₀ of ≤ 2 µM. Thus, the ingredients are powerful antioxidants and do usually show severe degradation in standard emulsions comprising water. In a preferred embodiment of the invention the cosmetic or therapeutic active exhibits an LDL-oxidation inhibition value IC₅₀ of ≥0,001 µM ≤ 5 µM, preferably of ≥ 0,01 µM ≤ 2 µM. These substance are effectively protected in the inventive polyol-in-oil-composition and do show significant protective effects on the skin even at low concentrations. The LDL-oxidation inhibition value IC₅₀ can be assessed according to McDowell IF et al. "A rapid method for measurement of the susceptibility to oxidation of low-density lipoprotein "; Ann. Clin. Biochem. 1995 Mar;32 (Pt 2):167-74.

A composition is furthermore within the scope of the invention, wherein the cosmetic or therapeutic active is ascorbic acid. Ascorbic acid can effectively be dissolved using the inventive polyol-in-oil-composition and exhibits an extraordinary stability profile. No discoloration of the polyol-in-oil-composition can be detected even at elevated storage temperatures. Without being bound by the theory this is achievable due to the low water activity in the polyol-in-oil-composition and the effective incorporation of the ascorbic acid in the emulsifier coated droplets, which is additionally shielding the ascorbic acid. In a preferred embodiment of this invention also a combination of ascorbic acid and Vitamin E can be incorporated. In addition, a combination of Vitamin C (ascorbic acid) and plant derived polyphenols has been proven to yield optically clear compositions. Furthermore, such mixture has been shown to be very stable in the inventive composition. Such stability in the composition might also be based on the pH-characteristics of the combined polyphenol/ascorbic acid in combination with the inventive polyol-in-oil delivery system.

Furthermore, a composition is within the scope of the invention, wherein additionally water is present in an amount of ≥ 1,0 weight-% and ≤ 10 weight-% and the composition comprises a polyol-in-oil-in-water (POW) structure. It was surprisingly found that the polyol-in-oil-compositions are effectively able to protect sensitive ingredients even in the presence of water. Without being bound by the theory this is possible, because the actives are incorporated to a large extent within the emulsifier coated droplets, which do anyhow form the larger volume amount of the composition. Thus, the actives are effectively shielded from contact with water, leading to a higher overall stability of the active.

Within a further object of the inventive composition the composition turbidity is ≥ 0,01 NTU and ≤ 50 NTU according to DIN EN ISO 7027. Favorably, it is possible to achieve optically clear or only slightly opaque polyol-in-oil-compositions by using the inventive composition. Without being bound by the theory this is achievable, because the inventive composition results in the formation of emulsifier coated droplets, having sizes small enough to prevent light scattering. In addition, it is possible to readily dissolve the actives in the chosen oil/emulsifier mixture. This is in contrast to other compositions, wherein the actives are just coarsely dispersed. Higher NTU values are not within the scope of the invention, because in that case the overall composition becomes too translucent. This behavior could affect the consumer acceptance. The NTU-values of the composition can be assessed according to known methods in the art, for in-stance according to DIN EN ISO 7027.

Also a process for the production of the inventive composition is within the scope of the invention , wherein in step
1) a low molecular weight polyol is heated to a temperature ≥ 50 °C and ≤ 100 °C,
2) a cosmetic or therapeutic active is dissolved into the heated low molecular weight polyol of step 1) by stirring until a clear solution is obtained
3) the composition of step 2) is added to a mixture of at least one polyether-free emulsifier and at least one oil component by stirring under laminar elongational flow conditions. By using such process it is possible to obtain polyol-in oil emulsion comprising a high very active content and a very narrow droplet size distribution, resulting in optically clear products. Furthermore, the emulsions are stable and no coalescence occurs, resulting in storage stable, optically clear emulsion. In addition, especially sensitive actives are protected, resulting in a superior storage stability of the active. If necessary, for instance in order to regulate the emulsion viscosity NaCl can be added in the low molecular weight polyol phase. The temperature of the overall process steps 1) - 3) can be in the range of ≥ 50 °C and ≤ 100 °C, preferably ≥ 60 °C and ≤ 85 °C. Such temperature range may ease the dissolution of the actives and may additionally favour a uniform droplet formation.

An optically clear solution is obtained in step 2) if no particles are visible in the composition upon visual inspection. Furthermore, a uniform droplet size distribution is obtainable by application of a mixing procedure, just using laminar elongational flow conditions. This is in contrast to turbulent mixing procedures, wherein only less homogenous droplet size distributions can be obtained. Without being bound by the theory the laminar elongational flow conditions might form in a first step worm-like droplets, which exhibit a spontaneous breakdown into uniform droplets. Therefore, a homogenous size distribution can be achieved using the inventive process.

A further characteristic of the invention may further include a process, wherein in step 2) additionally polyvinylpyrrolidone or polyvinylcaprolactam is added. It has surprisingly been found, that the addition of polyvinylpyrrolidone or polyvinylcaprolactam into the composition may further add some benefits. On the one hand composition features like the viscosity can be influenced by the addition of such substances, which in turn might also affect the storage stability of the composition. On the other hand such substances might also be able to influence the dermal delivery (or the uptake) of the actives. Therefore, also the penetration profile of the active can be tailored by using such penetration enhancer. The concentration of the polyvinylpyrrolidone or polyvinylcaprolactam may be ≥ 0,5 weight-% and ≤ 15 weight-%, preferably ≥ 2,5 weight-% and ≤ 12 weight-% and further preferred ≥ 5 weight-% and ≤ 10 weight-%. Such concentration may help to achieve a very good penetration profile of the active.

In another aspect of the inventive process the shear-rate in step 2) and 3) is ≥ 10 1/s and < 100000 1/s. Such shear-rate range may help to limit the stirring to laminar elongational flow, which in turn may result in the formation of a very uniform droplet size distribution.

A preferred characteristic of the inventive process may comprise an optional step 4), wherein the mixture of step 3) is combined under low-shear mixing with a water phase forming a polyol in oil in water emulsion. Optional step 4) may be performed either directly after the process steps 1) - 3) in the same or in a separate vessel or different compartment. This may also include the formation of the polyol-in-oil-in-water (POW)-emulsion directly before the application, for instance in a home-use treatment. The latter process is able to provide a POW-emulsion, including the application of a defined water content to the skin, but also minimizes the time, wherein the polyol-in-oil-composition (including the active) is in contact with water. This short contact time might reduce the necessity to incorporate preservatives, which in turn is helpful with respect to the dermatological acceptance of the formulation. In a preferred embodiment of the invention the weight ratio between the water and the polyol-in-oil-composition can be ≥1:100 and ≤ 1:1, preferably ≥1:10 and ≤ 1:1. Higher water concentrations are not within the scope of the invention, because this might lead to accelerated active degradation and hindrance of the active penetration. Lower water concentrations will not lead to a homogenous POW.

In addition, it is within the scope of the invention to disclose a use of the inventive composition in a dermatological, pharmacological or cosmetic skin-care-treatment. The disclosed polyol-in-oil-compositions are able to safely incorporate highly active ingredients and assure sufficient storage stability. Due to the fact that these emulsions might be formulated at zero or very low water contents the formulation can be very clean, avoiding possible allergens like preservatives or scents. In addition, due to the polyol, emulsifier and oil combination an effective delivery of the active in the skin is achievable. Therefore, very skin-friendly and effective compositions are formulated, which are very suitable in the above mentioned application fields.

Furthermore, the inventive composition may be used in a skin-care-treatment, wherein the skin-care-treatment is a whitening or sun-protection treatment. Especially in the area of whitening and sun-protection application the use of highly effective ingredients is necessary. In the first application field this is mandatory, because the melanin in extremely stable and located in the deeper layers of the dermis. Therefore, besides highly active ingredients also ef fective vehicles, which allow the penetration of the actives into deeper skin-layers are necessary. For sun-protection applications it is necessary to assure the stability of the ingredients, here for instance radical-scavengers, in order to reliably protect the skin from sunburns.

Also a kit-of parts is within the scope of the invention comprising a dual-chamber dispenser comprising at least a) one chamber comprising the inventive polyol-in-oil-composition and b) one chamber comprising an aqueous composition comprising a water content greater than or equal to 70 weight% and smaller than or equal to 100 weight%. Due to the formulation of the inventive polyol-in-oil-compositions it is possible to generate POWs, directly before the application by using a dual chamber dispenser. This is advantageous, because most of the time the active is protected in the polyol-in-oil-composition having no direct contact with the water. Only right before the application the water is added and the POW generated. The additional water content might be beneficial for the skin feel of the overall composition and might also contribute to a slight disturbance of the lipid skin layer, thus enhancing the penetration of the active. Also additional sensory benefits like a slight cooling effect may be achieved by the additional water content. The water content of the aqueous composition in the second chamber can be greater than or equal to 80 weight% and smaller than or equal to 100 weight% and preferably greater than or equal to 90 weight% and smaller than or equal to 100 weight%.

With respect to additional advantages and features of the previously described kit-of parts it is explicitly referred to the disclosure of the inventive process, the inventive use and the inventive composition. In addition, also aspects and features of the inventive kit-of parts shall be deemed applicable and disclosed to the inventive process, the inventive use and the inventive composition. Furthermore, all combinations of at least two features disclosed in the claims and/or in the description are within the scope of the invention unless otherwise explicitly indicated. Other variations to be disclosed embodiments can be understood and effected by those skilled in the art in practicing the claimed invention, from a study of the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

### Examples

Following formulations comprising Vitamin C (ascorbic acid) as active ingredient were prepared.

| | **1** | **2** | **3** | **4** | **5** |
|---|---|---|---|---|---|
| | | | | | |
| Phase A | [%wt] | [%wt] | [%wt] | [%wt] | [%wt] |
| 1,2 Propylenglycol | 73,05 | 73,05 | 81,75 | 76,75 | 73,05 |
| NaCl | 0,25 | 0,25 | 0,25 | 0,25 | 0,25 |
| | | | | | |
| Phase B | | | | | |
| Polyvinylpyrrolidone | 0,00 | 0,00 | 0,00 | 0,00 | 7,70 |
| Polyvinylcaprolactam | 7,70 | 7,70 | 0,00 | 0,00 | 0,00 |
| Ascorbic Acid | 10,00 | 10,00 | 8,00 | 10,00 | 10,00 |
| | | | | | |
| Phase C | | | | | |
| Amodimethicone Glycerocarbamate | 4,00 | 4,00 | 4,00 | 4,00 | 4,00 |
| Cyclopentasiloxane | 5,00 | 0,00 | 0,00 | 9,00 | 5,00 |
| Isohexadecane | 0,00 | 5,00 | 6,00 | 0,00 | 0,00 |
| | | | | | |
| | **100,00** | **100,00** | **100,00** | **100,00** | **100,00** |

The inventive optically transparent polyol-in-oil-compositions can be processed, according to the following method:
Processing:
   Step 1) Heat Phase A to 70°C and stir until NaCl is dissolved.
   Step 2) Add powder Phase B to Phase A and stir until a clear solution is obtained.
   Sep 3) Add combined Phases A + B to Phase C whilst mixing under laminar elongational flow conditions.

An optically transparent polyol-in-oil-composition is obtained and assessment of the composition particle size (e.g. via MALLS Multi-Angle laser light scattering) reveals a very uniform mono-modal droplet size distribution comprising small droplet sizes in the range of 200 - 400 nm, i.e.

| Example | **1** | **2** | **3** | **4** |
|---|---|---|---|---|
| particle size [µm] | 0,230 | 0,250 | 0,236 | 0,322 |
| standard deviation [µm] | 0,0671 | 0,0688 | 0,0728 | 0,0755 |

All compositions do exhibit very good storage stability of the composition itself and especially the active component. The compositions remain optically clear and within a storage period of 420 days at room temperature no Vitamin C degradation can be detected.

### Dermatological Testing

Several dermatological tests have been performed using the inventive optically transparent polyol-in-oil-composition.

### A). Dermatological Patch test

A dermatologic patch test was performed using formula 1. No adverse effects can be detected in a patch test (n= 20).

### B). Age spot treatment

2 mg/cm² of the test product comprising 10% Vitamin C were applied on a specific area once a day and for four weeks. After 30 days a color reduction of 20% was detectable. The determination of the color reduction was done by chromatography.

### C). Reduction of lipid-peroxide formation

Radical formation on the skin induced by oxidation of skin lipids such as squalene was tested under UVA radiation (10 J/cm²). An optically transparent polyol-in-oil-composition comprising 10 % Vitamin C is able to reduce the formation of lipid oxidation species to approx 30 % compared to the untreated sample.

## Claims

1. Optically transparent polyol-in-oil-composition, **characterised in that** the composition comprises
a) ≥ 40 weight-% and ≤ 90 weight-% of at least one low molecular weight polyol,
b) ≥ 1,0 weight-% and ≤ 25 weight-% of at least one cosmetic or therapeutic active,
c) ≥ 1,0 weight-% and ≤ 10 weight-% of at least one polyether-free emulsifier
d) ≥ 2,0 weight-% and ≤ 25 weight-% of at least one oil component,
wherein the emulsion comprises emulsifier coated droplets in a size range of ≥ 20 nm and ≤ 1000 nm.

2. Composition according to any of the preceding claims, wherein the emulsifier is a polyether-free amino-functional silicon-derivative.

3. Composition according to any of the preceding claims, wherein the emulsifier is amodimethicone glycerocarbamate.

4. Composition according to any of the preceding claims, wherein additionally a low molecular weight alcohol is present in an amount of ≥ 0,5 weight-% and ≤ 15 weight-%.

5. Composition according to any of the preceding claims, wherein additionally a polyvinylpyrrolidone and/or a polyvinylcaprolactam is present in an amount of ≥ 0,5 weight-% and ≤ 15 weight-%.

6. Composition according to any of the preceding claims, wherein the cosmetic or therapeutic active is selected from the group comprising ascorbic acid, tocopherol, retinol, beta-carotene, polyphenols, trolox, quercetin, rutin, myricetin, kaempferol, catechin, epigallocatechin, epicatechin, resveratrol, red wine extract, white wine extract, grape extract, black tea extract, green tea extract, white tea extract.

7. Composition according to any of the preceding claims, wherein cosmetic or therapeutic active is ascorbic acid.

8. Composition according to any of the preceding claims, wherein additionally water is present in an amount of ≥ 1,0 weight-% and ≤ 10 weight-% and the composition comprises a polyol-in-oil-in-water (POW) structure.

9. Composition according to any of the preceding claims, wherein the composition turbidity is ≥ 0,01 NTU and ≤ 50 NTU according to DIN EN ISO 7027.

10. Process for the production of a composition according to any of the preceding claims,
wherein in step
1) a low molecular weight polyol is heated to a temperature ≥ 50 °C and ≤ 100 °C
2) a cosmetic or therapeutic active is dissolved into the heated low molecular weight polyol of step 1) by stirring until a clear solution is obtained
3) the composition of step 2) is added to a mixture of at least one polyether-free emulsifier and at least one oil component by stirring under laminar elongational flow conditions.

11. Process according to according to claim 10, wherein in step 2) additionally polyvinylpyrrolidone or polyvinylcaprolactam is added.

12. Process according to any of the claims 10-11, wherein in an optional step 4) the mixture of step 3) is combined under low-shear mixing with a water phase forming a polyol in oil in water emulsion.

13. Use of the composition according to any of the claims 1-10 in a dermatological, pharmacological or cosmetic skin-care-treatment.

14. Use according to claim 13, wherein the skin-care-treatment is a whitening or sun-protection treatment.

15. Kit-of parts comprising a dual-chamber dispenser comprising at least
a) one chamber comprising a polyol-in-oil-composition according to any of the claims 1-9 and
b) one chamber comprising an aqueous composition comprising a water content greater than or equal to 70 weight% and smaller than or equal to 100 weight%.
